# EUROPÄISCHE PATENTSCHRIFT

(11) **EP 2 823 800 B1**
(45) Veröffentlichungstag und Bekanntmachung des Hinweises auf die Patenterteilung: **21.08.2019**
(21) Anmeldenummer: 13175925.0
(22) Anmeldetag: 10.07.2013
(51) Int. Cl.: A61K 6/00

(54) **System zur Füllung eines Zahnwurzelkanals und zur Überdeckung von Pulpa**
System for filling a root canal of a tooth and for covering pulpa
Système destiné à remplir un canal de racine dentaire et à recouvrir la pulpe

(43) Veröffentlichungstag der Anmeldung: 14.01.2015
(73) Patentinhaber: Coltène/Whaledent AG, 9450 Altstaetten (CH)
(72) Erfinder: Schlüter, Martin, 88239 Wangen (DE); Ezeh, Benjamin, 6830 Rankweil (AT); Mannschedel, Werner, 89129 Langenau (DE)
(74) Vertreter: Hepp Wenger Ryffel AG

(56) Entgegenhaltungen:
- EP-A2- 0 864 312
- DE-A1- 10 021 605
- DE-A1-102007 029 640
- US-A- 6 126 446
- US-A1- 2011 281 241

## Beschreibung

Die Erfindung betrifft das Gebiet der zahnmedizinischen Wurzelkanalversorgung, insbesondere Zusammensetzungen zum Füllen von Wurzelkanälen und zur Überdeckung von Pulpa.

Zur klassischen Behandlung einer irreversiblen Schädigung des Zahnmarks wird die erkrankte Pulpa mechanisch aus dem Wurzelkanal entfernt, der Wurzelkanal gereinigt und ausgebohrt, mit einem elastisch-plastischen Element oder einem anderen Füllmaterial gefüllt und danach versiegelt. Ein ideales Wurzelkanalfüllmittel sollte das periapikale Gewebe nicht reizen, die Wurzelkanäle lateral und vertikal dicht verschliessen, volumenbeständig sein und im Wurzelkanal jedenfalls nicht schrumpfen. Zum Stand der Technik sei beispielsweise auf Friedman et al. in J. Dent. Res., 54 1975, 921-925, DE 10 2007 029640, US 2011/281241, Briseno in Philipp J., 7(2) 1990, 65-73 und US 4,632,977 verwiesen. *Briseno* beschreibt als Wurzelkanalfüllmaterialien unter anderem halbfeste Zemente auf Kunstharz-Basis, Zinkoxid-Eugenol-Basis, Calciumhydroxid-Basis und Glasionomer-Basis. Die US 4,632,977 schlägt Füllmaterialien auf *trans*-Polyisopren-Basis vor, beispielsweise auf Basis von Guttapercha oder Balata. Im Handel sind solche Zusammensetzungen bspw. als Guttapercha-Formkörper, sogenannte Guttapercha-Spitzen, erhältlich. Üblicherweise enthalten sie etwa 20 Gew.-% Guttapercha als Matrix, 60 bis 75 Gew.-% Zinkoxid als Füllstoff, 1 bis 17 Gew.-% Schwermetallsulfate als Röntgenkontrastmittel und 3 bis 4 Gew.-% Wachse und Harze als Weichmacher. Solche Guttapercha-Zusammensetzungen sind thermoplastisch und können daher im fliessfähigen Zustand gut zur Anlage an das Innere des Wurzelkanals gebracht werden.

Aus EP 864 312 und DE 100 21 605 ist ein System zur Füllung von Zahnwurzelkanälen bekannt, welches auf einer additionsvernetzenden Silikonzusammensetzung basiert. In eine solche Zusammensetzung kann zusätzlich ein Wurzelkanalstift auf Polyisopren-Basis (bspw. Guttapercha) eingebettet werden. Die Handhabbarkeit eines solchen Systems ist jedoch nicht unter allen Bedingungen optimal.

Aus EP 951 895 bzw. US 6 126 446 ist ein System bekannt, bei welchem ein Guttaperchapulver direkt in einen Sealer (bspw. auf Basis von Silikonmaterialien) eingearbeitet ist. Hierdurch konnte die Handhabbarkeit und die Reproduzierbarkeit der Ergebnisse des Systems aus Sealer und Guttapercha bereits verbessert werden.

Ebenfalls beschrieben wurde die Applikation von mineralischem Trioxid-Aggregat in die präparierte Wurzelspitze, wobei der Wurzelkanal anschliessend konventionell mit Guttaperacha und Sealer verschlossen wurde (Schweiz. Monatsschr. Zahnmed., Vol. 114: 3/2004, S. 223-230). Nachteilig hierbei sind jedoch die umständliche Handhabung von mehreren Materialien in mehreren Schritten sowie die damit verbundenen Unannehmlichkeiten für den Patienten aufgrund mehrerer notwendiger Sitzungen.

Es ist eine Aufgabe der Erfindung, die Handhabbarkeit und den Behandlungserfolg von Systemen zur Wurzelkanalfüllung weiter zu verbessern.

Diese Aufgabe wird gelöst durch ein System zur Füllung eines Zahnwurzelkanals und zur Überdeckung von Pulpa, auf Basis eines Silikonmaterials gemäß Anspruch 1.

Unter dem Begriff *"System"* werden im Rahmen der Erfindung sowohl ein- als auch zweikomponentige Darreichungsformen der Wurzelfüllungszusammensetzungen verstanden. Bei einer einkomponentigen Zusammensetzung liegen die zur Silikonzusammensetzung vernetzenden Bestandteile bereits vorgemischt vor; die Vernetzung erfolgt dann über den Zutritt von Feuchtigkeit. Bei zweikomponentigen Systemen liegen die zur Vernetzungsreaktion befähigten Bestandteile der Zusammensetzung getrennt, jedoch zur gemeinsamen Verwendung hergerichtet, vor. Die Vernetzung solcher zweikomponentigen Systeme erfolgt in der Regel unabhängig vom Zutritt von Feuchtigkeit.

Die vorgenannten Systeme des Stands der Technik basierend auf Silikonmaterialien sind inert; sie unterstützen oder beschleunigen insbesondere auch nicht die Neubildung von Hartsubstanz. Es wurde nun gefunden, dass die zusätzliche Anwesenheit von MₓO_{y}, wobei MₓO_{y} ausgewählt ist aus der Gruppe bestehend aus CaO, BaO, MgO, Na₂O, K₂O, SrO, bzw. einer MₓO_{y}-haltigen Verbindung (insbesondere einer eine CaO/SiO₂-haltige Verbindung) in einem Silikonmaterial die Bildung von Hartsubstanz signifikant fördert. Bevorzugt liegt der Gewichtsanteil des MₓO_{y} (insbesondere des CaO) bzw. der vorgenannten MₓO_{y}-haltigen Verbindungen im Bereich von 3 Gew.-% bis 80 Gew.-%. Dass dies die Verarbeitbarkeit und die resultierenden Dichtungseigenschaften des Silikonmaterials an sich zudem nicht konterkariert, war ebenfalls nicht erwartbar. Entgegen der Erwartung hat diese Bildung von Hartsubstanz einen positiven Effekt auf die Dichtungseigenschaften. Insbesondere können Risse und Schwachstellen im Dentin besser ausgeglichen werden. Zudem können unerwünschte, beispielsweise braune Verfärbungen vermieden werden.

Des Weiteren kann mit dem erfindungsgemässen System eine überraschenderweise sehr zuverlässige und dauerhafte Pulpaüberdeckung erreicht werden.

Die MₓO_{y}-haltige Verbindung weist eine mittlere Partikelgrösse (gewogenes Mittel) von < 100 µm, bevorzugt im Bereich von 0,01 bis 90µm, weiter bevorzugt von 0,1 bis 90 µm, besonders bevorzugt im Bereich von 1 bis 60 µm auf. Bei derartigen Partikelgrössen lässt sich die MₓO_{y}-haltige Verbindung sehr gut in die Silikonzusammensetzung einarbeiten, und auch die vorstehend beschriebenen Effekte der Neubildung von Hartsubstanz sind gut ausgeprägt.

Das erfindungsgemäße System weist weiter mindestens ein Polymer auf Basis von Isopren auf. Das Polymer auf Basis von Isopren ist hierbei ausgewählt aus der Gruppe bestehend aus Polyisopren, insbesondere trans-1,4-Polyisopren; Guttapercha; Balata; sowie Mischungen davon. Das Polymer auf Basis von Isopren weist hierbei eine mittlere Partikelgrösse (gewogenes Mittel) von < 100 µm auf, insbesondere im Bereich von 1 bis 60 µm, bevorzugt von 2 bis 45 µm, weiter bevorzugt von 5 bis 30 µm, besonders bevorzugt von 10 bis 30 µm.

Mit derartigen Zusammensetzungen bspw. auf Basis von additionsvernetzenden Silikonen und Guttaperchapulver wurde gefunden, dass sie die vorteilhaften Eigenschaften der Zusammensetzungen von EP 951 895 nach wie vor aufweisen, jedoch zusätzlich die Bildung von Hartsubstanz gefördert wird, wie vorstehend beschrieben.

Besonders bevorzugte Varianten der Erfindung betreffen additionsvernetzende Silikonsysteme mit getrennt vorliegende Komponenten A und B, wobei
- die Komponente A wenigstens ein oder mehrere Silikonöl(e) mit mindestens zwei Si-H-Gruppen enthält oder aus diesen besteht;
- die Komponente B wenigstens ein oder mehrere Silikonöl(e) mit mindestens zwei Vinylgruppen enthält oder aus diesen besteht.

Optional kann mindestens eine der Komponenten A oder B einen Katalysator für die Additionsreaktion von Si-H-Gruppen mit Vinylgruppen enthalten.

Die Vernetzung des Silikonmaterials erfolgt bei diesen Ausführungsformen der Erfindung über eine Hydrosilylierungsreaktion; schematisch wie folgt:

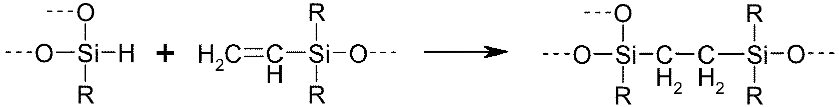

R bezeichnet hierbei beliebige Alkylreste, die gleich oder verschieden sein können. In besonders bevorzugten Ausführungsformen der Erfindung bezeichnet R eine Methylgruppe; diese Systeme basieren also auf Methylhydrosilanen und Dimethylsiloxanen.

Als Katalysatoren für Hydrosilylierungsreaktionen werden bevorzugt die an sich bekannten Platinkatalysatoren eingesetzt.

Weiter bevorzugt kann eine geringfügige Expansion des Silikonmaterials vorgesehen sein, zum Beispiel durch eine kontrollierte Quellung oder durch eine teilweise dehydrierende Kopplung, schematisch wie folgt: 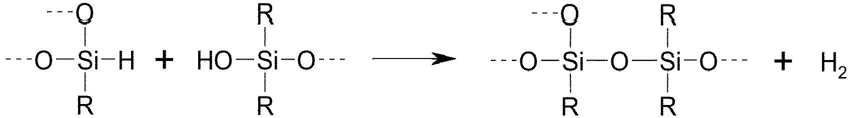 R bezeichnet hierbei wiederum beliebige Alkylreste, die gleich oder verschieden sein können (Ausführungsformen mit R = CH₃ sind jedoch bevorzugt). Durch die Entstehung von Wasserstoff erfolgt eine Schaumbildung bei der Vernetzung des Silikonmaterials, die über den Gehalt an hydroxylgruppenhaltigen Silikonölen vom Fachmann leicht mit Routineversuchen auf ein gewünschtes Ausmass eingestellt werden kann.

Optional kann als Katalysator für eine dehydrierende Kupplung ein hierfür an sich bekannter Katalysator wie bspw. Platinkatalysatoren, Zinkoctoat, Eisenoctoat, Dibutyldilaurylzinn oder eine Verbindung der allgemeinen Formel Sn(OOCR)₂ eingesetzt werden, wobei R einen Alkylrest bezeichnet.

Bei allen hierin beschriebenen zweikomponentigen Systemen gilt, dass
- das mindestens eine Polymer auf Basis von Isopren; und/oder
- die mindestens eine MₓO_{y}-haltige Verbindung sowohl in Komponente A als auch in Komponente B enthalten sein kann. Ganz besonders bevorzugt sind die genannten Bestandteile, soweit sie enthalten sind, auf die beiden Komponenten A und B gleichverteilt. Hierdurch kann eine besonders ausgewogene Verteilung dieser Bestandteile in der fertigen Mischung erreicht werden, ohne dass aufwändige Mischer vonnöten wären.

Alternativ kann das erfindungsgemässe System auch kondensationsvernetzend ausgebildet sein. Es enthält dann typischerweise:
i) wenigstens ein oder mehrere Silikonöl(e) mit mindestens zwei Si-OH-Gruppen;
ii) wenigstens ein oder mehrere Silikonöl(e) mit mindestens zwei funktionellen Gruppen, die optional unter Feuchtigkeitszutritt mit Si-OH-Gruppen reaktionsfähig sind.

Die Silikonöle können in diesen Ausführungsformen als ein- oder zweikomponentiges System bereitgestellt sein. Die praxisrelevantesten kondensationsvernetzenden Systeme umfassen als Bestandteil ii) Silikonöle mit funktionellen Silanendgruppen wie zum Beispiel

| | |
|---|---|
| Acetoxysilane: | 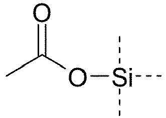 |
| Enoxysilane: | 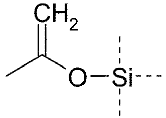 |
| Oximsilane: | 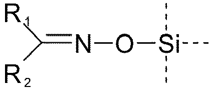 |
| | mit R₁ und R₂: H, Alkyl oder Aryl, unabhängig voneinander, gleich oder verschieden (jedoch nicht R₁ und R₂ gleichzeitig H). |
| Alkoxysilane: | 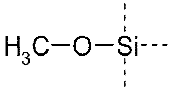 |
| | mit R₁ und R₂: Alkyl oder Aryl, unabhängig voneinander, gleich oder verschieden. |
| Aminosilane: | 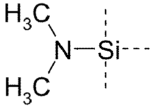 |
| | mit R₁ und R₂: H, Alkyl oder Aryl, unabhängig voneinander, gleich oder verschieden. |

Optional kann als Katalysator für eine Kondensationsvernetzung ein an sich bekannter Katalysator wie bspw. Zinkoctoat, Eisenoctoat, Dibutyldilaurylzinn oder eine Verbindung der allgemeinen Formel Sn(OOCR)₂ eingesetzt werden, wobei R einen Alkylrest bezeichnet.

Auf die Anwesenheit von Al₂O₃ (und dem häufig gemeinsam mit ihm vorkommenden Fe₂O₃) kann im Rahmen der Erfindung verzichtet werden. In Anbetracht einiger Studien zur vermeintlichen Neurotoxizität von Aluminium sind Al₂O₃-freie Zusammensetzungen im Rahmen der Erfindung besonders bevorzugt. Es hat sich überraschend gezeigt, dass die Anwesenheit von Al₂O₃ (insbesondere von C₃A (Tricalciumaluminat, (CaO)₃ x Al₂O₃), C₂AF (Dicalciumaluminat-ferrit, (CaO)₂ x Al₂O₃ x Fe₂O₃) und C₄AF (Tetracalciumaluminat-ferrit, (CaO)₄ x Al₂O₃ x Fe₂O₃), die alle typische Bestandteile von MTA sind) für das Funktionieren der Erfindung nicht entscheidend ist.

Die Anwesenheit von CaSO₄ x 2 H₂O (Gips) ist ebenfalls im Rahmen der Erfindung möglich, jedoch nicht zwingend.

Die Effekte der Erfindung sind noch nicht vollständig verstanden. Ohne an diese Erklärung gebunden zu sein wird jedoch derzeit angenommen, dass überraschenderweise die Silikonmaterialien diese Verbindungen nicht vollständig zu benetzen vermögen, so dass diese Materialien nach wie vor zumindest teilweise an der Oberfläche des Silikonmaterials derart exponiert sind, dass sie unter Einfluss von Körperflüssigkeit zur Bildung von Hartsubstanz (insbesondere Hydroxylapatit) Anlass geben.

Apatit ist die Kurz- und Sammelbezeichnung für eine Gruppe chemisch ähnlicher, aber nicht näher bestimmter Minerale, die dem Fachmann bekannt sind.

Auch erscheint es möglich, dass die Apatitbildung über aus der Zusammensetzung herausgelöste Ca²⁺, Ba²⁺, Mg²⁺, Sr²⁺, Na⁺ oder K⁺ Ionen erfolgt, welche durch H₃O⁺ Ionen auf der Oberfläche der Zusammensetzung ersetzt werden. Die sich hierdurch ergebenden SiOH Gruppen auf der Oberfläche der Zusammensetzung könnten die Bildung von Apatitkristallkeimen induzieren, was durch die Erhöhung des *ion activity product* (IAP) noch verstärkt werden könnte. Einmal gebildete Kristallkeime könnten spontan weiterwachsen aufgrund der in der Körperflüssigkeit im Überschuss vorhandene Calcium- und Phosphationen. Im Labor wird die Körperflüssigkeit simuliert; man spricht von *"*simulated body fluid", SBF (Kokubo et al., J. Biomed. Mater. Res., 24 (1990), 721-734). Die Ionenkonzentrationen von hier und im Weiteren verwendeter SBF sind wie folgt (im Vergleich zu menschlichem Blutplasma), wobei der pH-Wert auf 7,25 bei 36,5 °C eingestellt wird unter Verwendung von 50 mM Tris(hydroxymethyl)aminomethan und 45 mM HCl):

| | Konzentration [mmol/dm³] | |
|---|---|---|
| | SBF | Blutplasma |
| Na⁺ | 142,0 | 142,0 |
| K⁺ | 5,0 | 5,0 |
| Mg²⁺ | 1,5 | 1,5 |
| Ca²⁺ | 2,5 | 2,5 |
| Cl⁻ | 147,8 | 103,0 |
| HCO₃⁻ | 4,2 | 27,0 |
| HPO₄²⁻ | 1,0 | 1,0 |
| SO₄²⁻ | 0,5 | 0,5 |

Zwar ist die Erzeugung von Hydroxylapatit aus bspw. Calciumphosphat und Calciumhydroxid an sich bekannt (vergl. EP 367 808), jedoch unter stark basischen Bedingungen (pH > 11), summarisch wie folgt:

3 Ca (H₂PO₄)₂ × 2 H₂O + 7 Ca(OH)₂ → 2 Ca₅(PO₄)₃OH + 18 H₂O.

Derartig stark basische Reaktionsbedingungen sind *in vivo* jedoch offensichtlich unerwünscht. Zudem hat sich im Rahmen der Erfindung gezeigt, dass die Bildung von Hydroxylapatit erstaunlich rasch und unter milden Bedingungen in SBF, insbesondere SBF mit einem pH-Wert von bspw. 7,25, auch ausgehend von CaO bzw. SiO₂/CaO vonstatten geht.

Das erfindungsgemässe System enthält mindestens eine CaO/SiO₂-haltigen Verbindung(en) in Form eines Glases und/oder einer Glaskeramik. Bei einem solchen Glas kann es sich um eine binäre CaO/SiO₂-Zusammnsetzung, eine ternäre CaO/P₂O₅/SiO₂-Zusammensetzung oder auch um eine quaternäre SiO₂/CaO/P₂O₅/Na₂O-Zusammensetzung handeln; Mischungen der vorgenannten Zusammensetzungen sind selbstverständlich ebenfalls möglich.

Im Rahmen der Erfindung bevorzugte Gläser sind unter der Marke Bioglass® bekannt und erhältlich (sowie auch bspw. von Schott als sogenanntes bioaktives Glas); siehe hierzu Hench in J. Mater. Sci: Mater. Med (2006) 17, 967-978 (die Offenbarung dieses Dokuments bezüglich der Zusammensetzungen von Bioglass® wird hiermit durch Bezugnahme in diese Unterlagen aufgenommen). Im Rahmen der Erfindung geeignete bioaktive Gläser sind weiter beschrieben in Hupa, S. 3 ff. in Bioactive glasses: Materials, properties and applications, 2011, Woodhead Publishing Ltd., ISBN 1845697685 (die Offenbarung dieses Buchkapitels wird hiermit betreffend die Zusammensetzung bioaktiver Gläser durch Bezugnahme in diese Unterlagen aufgenommen).

Besonders bevorzugt kann das Glas im Rahmen der Erfindung ausgewählt werden aus den Gläsern i) und ii), welche die folgenden Bestandteile aufweisen (neben P₂O₅ mit einem Anteil tyischerweise zwischen 2 und 6 Gew.%, in Einzelfällen bis zu ca. 20 Gew.%):
i) 35 bis 65 mol%, insbesondere 35 bis 60 mol% SiO₂,
   10 bis 50 mol% CaO,
   5 bis 40 mol% Na₂O;
ii) 50,01 bis 65 mol% SiO₂,
   1 bis 9,99 mol% CaO,
   5 bis 40 mol% Na₂O.

Ganz besonders bevorzugt ist das Glas ausgewählt aus der Gruppe bestehend aus 45S5, 58S, S70C30, S53P4 sowie Mischungen davon.

Alle vorgenannten Systeme enthalten vorzugsweise auch ein Röntgenkontrastmittel, insbesondere ausgewählt aus Verbindungen der Gruppe bestehend aus Zink, Ytterbium, Yttrium, Gadolinium, Zirkonium, Strontium, Wolfram, Tantal, Niob, Barium, Bismut, Molybdän, Lanthan; Legierungen, Fluoriden, Sulfaten, Carbonaten, Wolframaten, Carbiden und Oxiden aller vorgenannten, besonders bevorzugt YbF₃ oder ZrO₂; organischen und anorganischen Iodverbindungen. Die jeweiligen Mengenanteile, in denen das Röntgenkontrastmittel der Zusammensetzung beizufügen ist um den gewünschten Kontrast zu erhalten, kann vom Fachmann leicht in Routineversuchen ermittelt werden.

Die erfindungsgemässen Systeme wie vorstehend beschrieben können vorportioniert in einer oder mehreren Kapsel(n), Kartusche(n) (insbesondere Doppelkammerspritzen) oder Schlauchbeutel(n) bereitgestellt werden. Dies gilt sowohl für ein- als auch zweikomponentige Systeme. Bei zweikomponentigen Systemen können die Komponenten A und B besonders bevorzugt in einer gemeinsamen Kapsel, Kartusche (insbesondere einer Doppelkammerspritze) oder Schlauchbeutel getrennt voneinander bereitgestellt werden, wobei die Kapsel in einen Dispenser einsetzbar ist. Die Handhabung ist in einer derartigen Darreichungsform in an sich bekannter Art und Weise vereinfacht. Die Bereitstellung der Komponenten A und B in getrennten Kapseln, Kartuschen (insbesondere Doppelkammerspritzen) oder Schlauchbeuteln ist aber selbstverständlich ebenfalls möglich.

Erfindungsgemässe Systeme wie vorstehend beschrieben können weiter mindestens einen Wurzelkanalstift enthalten, insbesondere auf Basis eines Polymers von Isopren. Diese Wurzelkanalstifte können in an sich bekannter Art und Weise mit den erfindungsgemässen Zusammensetzungen in einem Zahnwurzelkanal vergossen werden.

Weitere mögliche Bestandteile des erfindungsgemässen Systems wie vorstehend beschrieben sind Dispenser für Kapsel(n), Kartusche(n) (insbesondere Doppelkammerspritze(n)) oder Schlauchbeutel; Auftragsspitzen für fliessfähige Zusammensetzungen (zur Applikation der erfindungsgemässen Zusammensetzungen auf Basis eines Silikonmaterials); sowie auf einer Auftragsspitze verschiebbare Markierungshilfe zur Bestimmung der Wurzelkanaltiefe. Bei einer solchen Markierungshilfe kann es sich bspw. um einen elastischen Ring handeln, der auf der Auftragsspitze auf- und abgerollt werden kann.

Die Erfindung wird nachfolgend anhand von Ausführungsbeispielen und Figuren erläutert, ohne dass der Gegenstand der Erfindung auf diese Ausführungsformen zu beschränken ist. Hierbei zeigt:
- Fig. 1: REM-Aufnahme einer Pastille aus Silikon enthaltend eine CaO/SiO₂ Mischung, 14 d nach dem Einlegen in einen SBF-Puffer;
- Fig. 2: REM-Aufnahme der Pastille aus Silikon enthaltend bioaktives Glas, 14 d nach dem Einlegen in einen SBF-Puffer.

### Bioaktivität einer CaO/SiO₂ Mischung in Silikonmaterial

Folgende Mischung wurden hergestellt:

| | Gew.-% | Gew.-% | 4:1 |
|---|---|---|---|
| | **Base** | **Kat** | **Mischung** |
| Divinylpolydimethylsiloxane | 26,35 | 34,23 | 27,93 |
| Hydrogenmethylpolydivinylsiloxane | 9,25 | | 7,40 |
| Silikate | 3, 67 | 5,76 | 4,09 |
| Röntgenopaker | 14, 00 | 60,00 | 23,20 |
| Pigmente | 1,72 | | 1,38 |
| Guttaperchamischung | 20,00 | | 16,00 |
| Pt-Katalysator | | 0,01 | |
| CaO/SiO₂ Mischung* | 25,00 | | 20,00 |
| | 100,00 | 100,00 | 100,00 |

| | | | |
|---|---|---|---|
| * Als CaO/SiO₂ Mischung wurde eine Mischung aus CaO (65 Gew.%), SiO₂ (25 Gew.%) und Ca(HPO₄)₂ (10 Gew.%) eingesetzt. | | | |

Aus der erhaltenen Paste wurden Prüflinge in Form von Pastillen hergestellt. Hierfür wurde in einer Spaltringform mit einem Innendurchmesser von 20 mm und einer Höhe 1,5 mm eine Plastikfolie ausgebreitet und darauf ein Stück Zahnseide gelegt. Daraufhin wurden die oben beschriebenen Mischungen in die Spaltringform eingefüllt, eine zweite Plastikfolie aufgelegt und mit einer Glasplatte beschwert. Nach drei Stunden wurden die ausgehärteten Pastillen aus der Spaltringform daraufhin entnommen. Die somit erhaltenen Pastillen wurden dann an der Zahnseide hängend bei 37 °C in einen SBF-Puffer mit einem pH-Wert von 7,25 eingelegt.

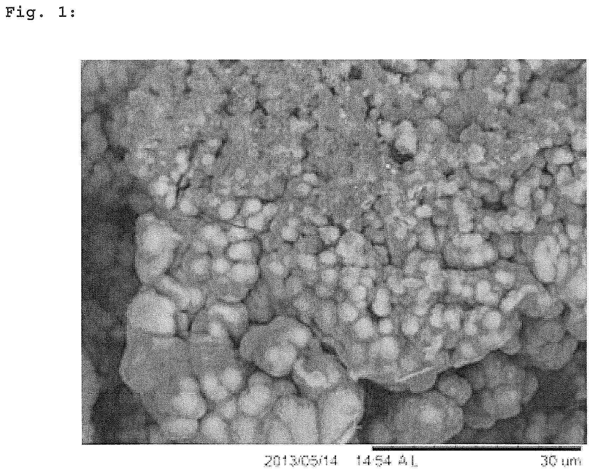

In Fig. 1 ist eine REM-Aufnahme nach 14 Tagen Einlegen in dem SBF Puffer zu sehen. Vor der Anfertigung der REM-Aufnahme wurde die Pastille jeweils mit Reinstwasser gespült und anschliessend getrocknet. Es ist ersichtlich, dass sich Kristalle auf der Oberfläche der Pastillen gebildet haben. Die kugelförmigen Kristalle haben die dem Fachmann bekannte Form von Apatit-Kristallen. EDX-Messungen sowie IR- und Raman-Spektroskopie haben die Apatitbildung ebenfalls bestätigt (Daten nicht gezeigt).

### Bioaktivität von Glasmaterialien in Silikonmaterial

Folgende Mischung wurden hergestellt:

| | Gew.-% | Gew.-% | 4:1 |
|---|---|---|---|
| | **Base** | **Kat** | **Mischung** |
| Divinylpolydimethylsiloxane | 26,35 | 34,23 | 27,93 |
| Hydrogenmethylpolydivinylsiloxane | 9,25 | | 7,40 |
| Silikate | 3,67 | 5,76 | 4,09 |
| Röntgenopaker | 14,00 | 60,00 | 23,20 |
| Pigmente | 1,72 | | 1,38 |
| Guttaperchamischung | 20,00 | | 16,00 |
| Pt-Katalysator | | 0,01 | |
| Bioaktives Glas** | 25,00 | | 20,00 |
| | 100,00 | 100,00 | 100,00 |

| | | | |
|---|---|---|---|
| ** Als bioaktives Glas wurde G018-144 (Schott AG) eingesetzt. | | | |

Aus der erhaltenen Paste wurden Prüflinge in Form von Pastillen hergestellt. Hierfür wurde in einer Spaltringform mit einem Innendurchmesser von 20 mm und einer Höhe 1,5 mm eine Plastikfolie ausgebreitet und darauf ein Stück Zahnseide gelegt. Daraufhin wurden die oben beschriebenen Mischungen in die Spaltringform eingefüllt, eine zweite Plastikfolie aufgelegt und mit einer Glasplatte beschwert. Nach drei Stunden wurden die ausgehärteten Pastillen aus der Spaltringform daraufhin entnommen. Die somit erhaltenen Pastillen wurden dann an der Zahnseide hängend bei 37 °C in einen SBF-Puffer mit einem pH-Wert von 7,25 eingelegt.

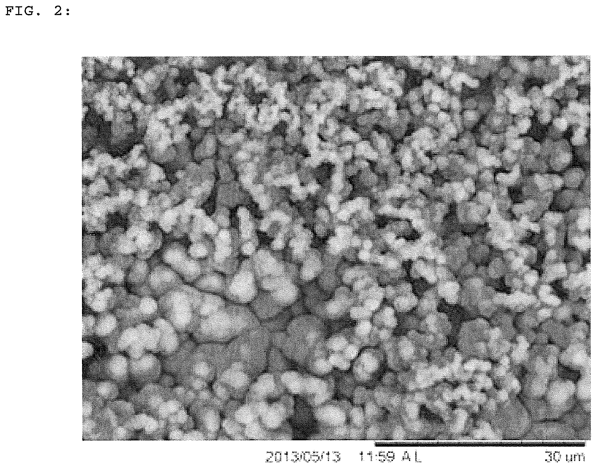

In Fig. 2 ist eine REM-Aufnahme nach 14 Tagen Einlegen in dem SBF Puffer zu sehen. Vor der Anfertigung der REM-Aufnahme wurde die Pastille jeweils mit Reinstwasser gespült und anschliessend getrocknet. Es ist ersichtlich, dass sich Kristalle auf der Oberfläche der Pastillen gebildet haben. Die kugelförmigen Kristalle haben die dem Fachmann bekannte Form von Apatit-Kristallen. EDX-Messungen sowie IR- und Raman-Spektroskopie haben die Apatitbildung ebenfalls bestätigt (Daten nicht gezeigt).

## Patentansprüche

1. System zur Füllung eines Zahnwurzelkanals und zur Überdeckung von Pulpa, auf Basis eines Silikonmaterials, umfassend entweder
- getrennt vorliegende Komponenten A und B, wobei
- die Komponente A wenigstens ein oder mehrere Silikonöl(e) mit mindestens zwei Si-H-Gruppen enthält oder aus diesen besteht;
- die Komponente B wenigstens ein oder mehrere Silikonöl(e) mit mindestens zwei Vinylgruppen enthält oder aus diesen besteht;
- insbesondere mindestens eine der Komponenten A oder B einen Katalysator für die Additionsreaktion von Si-H-Gruppen mit Vinylgruppen enthält; oder umfassend
-
i) wenigstens ein oder mehrere Silikonöl(e) mit mindestens zwei Si-OH-Gruppen;
ii) wenigstens ein oder mehrere Silikonöl(e) mit mindestens zwei funktionellen Gruppen, die optional unter Feuchtigkeitszutritt mit Si-OH-Gruppen reaktionsfähig sind;
wobei die Silikonöle als ein- oder zweikomponentiges System bereitgestellt sind;
wobei das System weiter umfasst
- mindestens eine MₓO_{y}-haltige Verbindung, wobei MₓO_{y} eine CaO/SiO₂-haltige Verbindung ist, nämlich ein Glas und/oder eine Glaskeramik, wobei das Glas insbesondere ausgewählt ist aus der Gruppe bestehend aus binären CaO/SiO₂-Zusammensetzungen; ternären CaO/P₂O₅/SiO₂-Zusammensetzungen und quaternären SiO₂/CaO/P₂O/Na₂O-Zusammensetzungen;
wobei die mindestens eine MₓO_{y}-haltige Verbindung eine mittlere Partikelgrösse (gewogenes Mittel) von < 100 µm, bevorzugt im Bereich von 0,01 bis 90 µm, weiter bevorzugt von 0,1 bis 90 µm, besonders bevorzugt im Bereich von 1 bis 60 µm aufweist;
wobei das System weiter mindestens ein Polymer umfasst, das ausgewählt ist aus der Gruppe bestehend aus Polyisopren, insbesondere *trans*-1,4-Polyisopren Guttapercha; Balata; sowie Mischungen davon,
mit einer mittleren Partikelgrösse (gewogenes Mittel) von < 100 µm, insbesondere im Bereich von 1 bis 60 µm, bevorzugt von 2 bis 45 µm, weiter bevorzugt von 5 bis 30 µm, bevorzugt von 10 bis 30 µm.

2. System gemäss Anspruch 1, **dadurch gekennzeichnet, dass** das mindestens eine Polymer; und/oder die mindestens eine MₓO_{y}-haltige Verbindung im zweikomponentigen Fall in beiden Komponenten enthalten ist/sind.

3. System gemäss einem der Ansprüche 1 und 2, **dadurch gekennzeichnet, dass** das Silikonmaterial ein Röntgenkontrastmittel enthält, insbesondere ausgewählt aus Verbindungen der Gruppe bestehend aus Zink, Ytterbium, Yttrium, Gadolinium, Zirkonium, Strontium, Wolfram, Tantal, Niob, Barium, Bismut, Molybdän, Lanthan; Legierungen, Fluoriden, Sulfaten, Carbonaten, Wolframaten, Carbiden und Oxiden aller vorgenannten, besonders bevorzugt YbF₃ oder ZrO₂; organischen und anorganischen Iodverbindungen.

4. System gemäss einem der Ansprüche 1 bis 3, vorportioniert in einer oder mehreren Kapsel(n), Kartusche(n) , insbesondere Doppelkammerspritze (n), oder Schlauchbeutel(n).

5. System gemäss einem der Ansprüche 1 bis 4, weiter enthaltend mindestens einen Wurzelkanalstift, insbesondere auf Basis eines Polymers von Isopren.

6. System gemäss einem der Ansprüche 1 bis 5, weiter enthaltend einen oder mehrere Bestandteile ausgewählt aus der Gruppe umfassend Dispenser für Kapsel(n), Kartusche(n), Doppelkammerspritzen oder Schlauchbeutel; Auftragsspitzen für fliessfähige Zusammensetzungen; auf einer Auftragsspitze verschiebbare Markierungshilfen zur Bestimmung der Wurzelkanaltiefe.

## Claims

1. System for filling a root canal of a tooth and for covering pulp based on a silicone material, comprising either
- components A and B present separately, wherein
- the component A comprises at least one or more silicone oil(s) with at least two Si-H groups or consists of these;
- the component B comprises at least one or more silicone oil(s) with at least two vinyl groups or consists of these;
- in particular at least one of the components A or B comprises a catalyst for the addition reaction of Si-H groups with vinyl groups; or comprising
-
i) at least one or more silicone oil(s) with at least two Si-OH groups;
ii) at least one or more silicone oil(s) with at least two functional groups which are optionally capable of reacting with Si-OH groups with admission of moisture;
wherein the silicone oils are made available as a one- or two-component system;
wherein the system furthermore comprises
- at least one MₓO_{y}-comprising compound, in which MₓO_{y} is a CaO/SiO₂-comprising compound, namely a glass and/or a glass ceramic, wherein the glass in particular is chosen from the group consisting of binary CaO/SiO₂ compositions, ternary CaO/P₂O₅/SiO₂ compositions and quaternary SiO₂/CaO/P₂O₅/Na₂O compositions;
wherein the at least one MₓO_{y}-comprising compound exhibits a mean particle size (weighted average) of < 100 µm, preferably in the range from 0.01 to 90 µm, more preferably in the range from 0.1 to 90 µm and particularly preferably in the range from 1 to 60 µm;
wherein the system furthermore comprises at least one polymer which is chosen from the group consisting of polyisoprene, in particular trans-1,4-polyisoprene, gutta-percha, balata;
and also mixtures thereof,
with a mean particle size (weighted average) of < 100 µm, in particular in the range from 1 to 60 µm, preferably from 2 to 45 µm, furthermore preferably from 5 to 30 µm and preferably from 10 to 30 µm.

2. System according to Claim 1, **characterized in that** the at least one polymer; and/or
the at least one MₓO_{y}-comprising compound;
is/are present in the two-component case in both components.

3. System according to one of Claims 1 and 2, **characterized in that** the silicone material comprises an X-ray contrast medium chosen in particular from compounds of the group consisting of zinc, ytterbium, yttrium, gadolinium, zirconium, strontium, tungsten, tantalum, niobium, barium, bismuth, molybdenum, lanthanum; alloys, fluorides, sulfates, carbonates, tungstates, carbides and oxides of all abovementioned elements, particularly preferably YbF₃ or ZrO₂; organic and inorganic iodine compounds.

4. System according to one of Claims 1 to 3, preproportioned in one or more capsule(s), cartridge(s), in particular double-chamber syringe(s), or tubular bag(s).

5. System according to one of Claims 1 to 4, furthermore comprising at least one root canal pin, in particular based on an isoprene polymer.

6. System according to one of Claims 1 to 5, furthermore comprising one or more constituents chosen from the group comprising dispensers for capsule(s), cartridge(s), double-chamber syringes or tubular bag(s); applicator tips for flowable compositions; and marking aids movable on an applicator tip for determining the root canal depth.

## Revendications

1. Système pour le remplissage d'un canal radiculaire et pour le recouvrement de pulpe, à base d'un matériau de silicone, comprenant :
- des composants A et B présents séparément, où
- le composant A contient au moins une ou plusieurs huiles de silicone contenant au moins deux groupes Si-H ou étant constitué par celles-ci ;
- le composant B contient au moins une ou plusieurs huiles de silicone contenant au moins deux groupes vinyle ou étant constitué par celles-ci ;
- notamment au moins un des composants A ou B contient un catalyseur pour la réaction d'addition de groupes Si-H avec des groupes vinyle ; ou comprenant
-
i) au moins une ou plusieurs huiles de silicone contenant au moins deux groupes Si-OH ;
ii) au moins une ou plusieurs huiles de silicone contenant au moins deux groupes fonctionnels, qui sont optionnellement réactifs avec les groupes Si-OH lors de l'entrée d'humidité ;
les huiles de silicone étant préparées sous la forme d'un système mono- ou bicomposant ;
le système comprenant en outre :
- au moins un composé contenant MₓO_{y}, MₓO_{y} étant un composé contenant CaO/SiO₂, à savoir un verre et/ou une vitrocéramique, le verre étant notamment choisi dans le groupe constitué par les compositions CaO/SiO₂ binaires ; les compositions CaO/P₂O₅/SiO₂ ternaires et les compositions SiO₂/CaO/P₂O₅/Na₂O quaternaires ;
ledit au moins un composé contenant MₓO_{y} présentant une taille de particule moyenne (moyenne en poids) < 100 µm, de préférence dans la plage allant de 0,01 à 90 pm, de manière davantage préférée de 0,1 à 90 µm, de manière particulièrement préférée dans la plage allant de 1 à 60 µm ;
ledit système comprenant en outre au moins un polymère, qui est choisi dans le groupe constitué par le polyisoprène, notamment le trans-1,4-polyisoprène, la gomme gutta-percha ; le gomme balata ; ainsi que leurs mélanges,
ayant une taille de particule moyenne (moyenne en poids) < 100 pm, notamment dans la plage allant de 1 à 60 µm, de préférence de 2 à 45 µm, de manière davantage préférée de 5 à 30 pm, de préférence de 10 à 30 µm.

2. Système selon la revendication 1, **caractérisé en ce que** ledit au moins un polymère et/ou ledit au moins un composé contenant MₓO_{y} sont contenus dans les deux composants dans le cas d'un système bicomposant.

3. Système selon l'une quelconque des revendications 1 et 2, **caractérisé en ce que** le matériau de silicone contient un agent de contraste radiographique, notamment choisi parmi les composés du groupe constitué par le zinc, l'ytterbium, l'yttrium, le gadolinium, le zirconium, le strontium, le tungstène, le tantale, le niobium, le baryum, le bismuth, le molybdène, le lanthane ; les alliages, les fluorures, les sulfates, les carbonates, les tungstates, les carbures et les oxydes de tous les éléments susmentionnés, de manière particulièrement préférée YbF₃ ou ZrO₂ ; les composés d'iode organiques et inorganiques.

4. Système selon l'une quelconque des revendications 1 à 3, préportionné dans une ou plusieurs capsules, cartouches, notamment seringues à chambre double, ou sachets tubulaires.

5. Système selon l'une quelconque des revendications 1 à 4, contenant en outre au moins un pivot canalaire, notamment à base d'un polymère d'isoprène.

6. Système selon l'une quelconque des revendications 1 à 5, contenant en outre un ou plusieurs constituants choisis dans le groupe comprenant les distributeurs pour capsule(s), cartouche(s), seringues à chambre double ou sachets tubulaires ; les pointes d'application pour compositions fluides ; les auxiliaires de marquage déplaçables sur une pointe d'application pour la détermination de la profondeur du canal radiculaire.
